# EUROPÄISCHE PATENTSCHRIFT

(11) **EP 0 857 477 B1**
(45) Veröffentlichungstag und Bekanntmachung des Hinweises auf die Patenterteilung: **21.07.1999**
(21) Anmeldenummer: 98101957.3
(22) Anmeldetag: 05.02.1998
(51) Int. Cl.: A61K 7/13

(54) **Haarfärbemittel**
Hair dye
Teinture pour les cheveux

(30) Priorität: 08.02.1997 DE 19704852
(43) Veröffentlichungstag der Anmeldung: 12.08.1998
(73) Patentinhaber: GOLDWELL GmbH, D-64297 Darmstadt (DE)
(72) Erfinder: Lorenz, Heribert, 64401 Gross-Bieberau (DE); Kufner, Frank, 64297 Darmstadt (DE)

(56) Entgegenhaltungen:
- EP-A- 0 615 743
- DE-A- 4 120 361

## Beschreibung

Die vorliegende Erfindung betrifft ein Haarfärbemittel auf Basis eines mit Peroxid reagierenden Entwickler-Kuppler-Systems, das ausdrucksvolle und dauerhafte Farbtöne liefert, die entweder als solche angewandt werden, oder, in Kombination mit weiteren Entwickler- und/oder Kupplersubstanzen, zur Erzielung weiterer Farbnuancen benutzt werden können.

Die nach wie vor in Haarfärbemitteln meist eingesetzten Entwicklersubstanzen sind 1,4-Diaminobenzol (p-Phenylendiamin) und 1-Methyl-2,5-diaminobenzol (p-Toluylendiamin). Die Verwendung dieser Substanzen ist insofern nicht völlig problemfrei, als sie bei extrem empfindlichen Personen in speziellen Fällen zu Hautsensibilisierungen führen können (bei sogenannten "Para-Allergikern").

Es wurde bereits versucht, dieses Problem durch Verwendung alternativer Entwicklersubstanzen zu lösen. Dies ist in beschränktem Umfang möglich durch den Einsatz von Tetraaminopyrimidin oder 2-(2,5-Diaminophenyl)ethanol (vgl. EP-A 7537 und EP-B 400 330); jedoch müssen dann erhebliche Abstriche in der Färbeintensität und den Variationsmöglichkeiten der verschiedenen Farbtöne hingenommen werden.

Eine weitgehend optimale Lösung des Problems, nämlich die Abwesenheit von Hautsensibilisierung einerseits und eine große Variationsbreite der Erzielung möglicher Farbnuancen andererseits, wird durch den in der EP-A 615 743 beschriebenen Einsatz von 2-(2'-Hydroxyethylamino)-5-aminotoluol bzw. dessen wasserlöslichen Salzen als Entwicklersubstanzen in Haarfärbemitteln erreicht.

Selbst dadurch bleiben jedoch noch farbtechnische Wünsche offen.

Die Erfindung geht daher von der Aufgabenstellung aus, ein Haarfärbemittel zu schaffen, das eine nichtsensibilisierende Entwicklersubstanz enthält und zur Herstellung variabler Farbtöne geeignet ist.

Diese Aufgabe wird dadurch gelöst, daß ein solches Haarfärbemittel ein mit Peroxid reagierendes Oxidationsfarbstoff-Vorprodukt enthält, das aus 2-Amino-5-diethylaminotoluol bzw. dessen wasserlöslichen Salzen besteht.

Es wurde dabei festgestellt, daß 2-Amino-5-diethylaminotoluol nicht nur in Kombination mit üblichen Kupplersubstanzen eingesetzt werden kann, sondern offensichtlich auch eine gewisse Kupplerfunktion ausübt.

So können in Kombination mit anderen Entwicklersubstanzen, ebenfalls überraschend, voll befriedigende Haarfärbungen hergestellt werden.

Bei Anwendung dieser Zusammensetzungen auf Basis einer üblichen Grundlage werden nach der Oxidation mit Peroxid sehr ausdrucksvolle Grundfärbungen erhalten, die durch Einsatz von Gemischen entsprechender Kupplersubstanzen zu anderen Farbnuancen variiert werden können.

Bevorzugte Kupplersubstanzen, die erfindungsgemäß in einem molaren Verhältnis von Entwicklersubstanz zu Kupplersubstanz zwischen etwa 1:8 und 8:1, insbesondere 1:5 bis 5:1, vorzugsweise 1 : 1 und etwa 2,5 : 1 verwendet werden, sind Resorcin, 2-Methylresorcin, 4-Chlorresorcin, 2-Amino-4-chlorphenol, 4-(N-Methyl)aminophenol, 2-Aminophenol, 3-Aminophenol, 2,4-Diaminophenol, 5-Amino-2-methoxyphenol, 1-Methyl-2-hydroxy-4-aminobenzol, 3-N,N-Dimethylaminophenol, 4-Amino-3-methylphenol, 5-Amino-2-methylphenol, 6-Amino-3-methylphenol,2,6-Dihydroxy-3,4-dimethylpyridin, 3-Amino-2-methylamino-6-methoxypyridin, 2-Amino-3-hydroxypyridin, 4-Aminodiphenylamin, 4,4'-Diaminodiphenylamin, 2-Dimethylamino-5-aminopyridin, 2,6-Diaminopyridin, 2,5-Diaminopyridin 1,3-Diaminobenzol, 1-Amino-3-(2'-hydroxyethylamino)benzol, 1-Amino-3-[bis(2'-hydroxyethyl)amino]benzol, 1,3-Diaminotoluol, α-Naphthol, 1,4-Diamino-2-chlorbenzol, 4,6-Dichlorresorcin, 4-Hydroxy-1,2-methylendioxybenzol, 1,5-Dihydroxynaphthalin, 1,7-Dihydroxynaphthalin, 2,7-Dihydroxynaphthalin, 1-Hydroxynaphthalin, 1,5-Diaminonaphthalin, 4-Hydroxy-1,2-methylendioxybenzol, 2,4-Diamino-3-chlorphenol, 1-Phenyl-3-methylpyrazolon-(5) und/oder 1-Methoxy-2-amino-4-(2'-hydroxyethylamino)benzol.

Bevorzugte Kupplersubstanzen sind insbesondere Resorcin, 2-Methylresorcin, 4-Chlorresorcin, 3-Aminophenol, 1-Methyl-2-hydroxy-4-aminobenzol, 2-Amino-4-β-hydroxyethylaminoanisol bzw. dessen wasserlösliche Salze und α-Naphthol.

Damit soll jedoch der Zusatz weiterer Kupplersubstanzen keineswegs ausgeschlossen sein.

Auch die Mitverwendung weiterer, an sich bekannter Entwicklersubstanzen ist möglich. Neben den bereits eingangs genannten sind hierbei insbesondere noch 4-Aminophenol, 5-Aminosalicylsäure, N,N-Bis(hydroxyethyl)-1,4-diaminobenzol, 3-(Diethylaminomethyl)-4-hydroxyanilin, und/oder Hydroxytriaminopyrimidine, wie sie aus der EP-B 467 026 bekannt sind, zu erwähnen.

Als solche werden insbesondere das aus der bereits erwähnten EP-B 467 026 bekannte 2,4,5-Triamino-6-hydroxypyrimidin und das 4,5,6-Triamino-2-hydroxypyrimidin eingesetzt, insbesondere auch als Sulfat-Salze.

Die Konzentration der Entwicklersubstanzen liegt zwischen etwa 0,05 und 5 %, vorzugsweise 0,1 und 4 %, insbesondere 0,25 bis 0,5 % und 2,5 bis 3 % Gew.-% der Gesamtzusammensetzung des Haarfärbemittels (ohne Oxidationsmittel), wobei sich die Angaben jeweils auf den Anteil an freier Base beziehen.

Die Kupplersubstanzen als Reaktionspartner der Entwicklersubstanz(en) liegen in den erfindungsgemäßen Haarfärbemitteln etwa im gleichen Anteil wie die Entwicklersubstanzen vor, d. h., also in Mengen von 0,05 bis 5,0 %, vorzugsweise 0,1 bis 4 %, insbesondere 0,5 bis 3 Gew.-% der Gesamtzusammensetzung (ohne Oxidationsmittel), wobei sich die Angaben jeweils auf den Anteil an freier Base beziehen.

Auch hier ist es, wie bereits erwähnt, möglich und zuweilen auch zweckmäßig, weitere bekannte Kupplersubstanzen mitzuverwenden, falls dies zur Erzielung bestimmter Farbnuancen erwünscht und erforderlich ist.

Die erfindungsgemäßen Zusammensetzungen können erwünschtenfalls auch sogenannte Nuanceure zur Feineinstellung des gewünschten Farbtones, insbesondere auch direktziehende Farbstoffe, enthalten.

Solche Nuanceure sind beispielsweise Nitrofarbstoffe wie 2-Amino-4,6-dinitrophenol, 2-Amino-4-nitrophenol, 2-Amino-6-chlor-4-nitrophenol, etc., vorzugsweise in Mengen von etwa 0,05 bis 2,5 %, insbesondere 0,1 bis 1 % Gew.-% der Farbzusammensetzung (ohne Oxidationsmittel).

Das 2-Amino-5-diethylaminotoluol gelangt vorzugsweise in Form seiner wasserlöslichen Salze, beispielsweise als Hydrochlorid oder Sulfat, zum Einsatz.

Die erfindungsgemäßen Haarfärbemittel können die in solchen Mitteln üblichen Grund- und Zusatzstoffe, Konditioniermittel, etc. enthalten, die dem Fachmann aus dem Stand der Technik bekannt und beispielsweise in der Monographie von K. Schrader, "Grundlagen und Rezepturen der Kosmetika", 2. Aufl. (Hüthig Buch Verlag, Heidelberg, 1989), S. 782 bis 815, beschrieben sind. Sie können als Lösungen, Cremes, Gele oder auch in Form von Aerosol-Präparaten vorliegen; geeignete Trägermaterial-Zusammensetzungen sind aus dem Stand der Technik hinreichend bekannt.

Zur Applikation wird das erfindungsgemäße Oxidationsfarbstoff-Vorprodukt mit einem Oxidationsmittel vermischt. Bevorzugtes Oxidationsmittel ist Wasserstoffperoxid beispielsweise in 2- bis 6-prozentiger Konzentration.

Es können jedoch auch andere Peroxide wie Harnstoffperoxid und Melaminperoxid eingesetzt werden.

Der pH-Wert des applikationsfertigen Haarfärbemittels, d. h. nach Vermischung mit Peroxid, kann sowohl im schwach sauren, d. h. einem Bereich von 5,5 bis 6,9, im neutralen als auch im alkalischen Bereich, d. h. zwischen pH 7,1 und 9,5 liegen.

### Grundlage

| | |
|---|---|
| Stearylalkohol | 8,0 (Gew.-%) |
| Kokosfettsäuremonoethanolamid | 4,5 |
| 1,2-Propandiolmono/distearat | 1,3 |
| Kokosfettalkoholpolyglykolether | 4,0 |
| Natriumlaurylsulfat | 1,0 |
| Ölsäure | 2,0 |
| 1,2-Propandiol | 1,5 |
| Na-EDTA | 0,5 |
| Natriumsulfit | 1,0 |
| Eiweißhydrolysat | 0,5 |
| Ascorbinsäure | 0,2 |
| Parfum | 0,4 |
| Ammoniak, 25%ig | 8,5 |
| Ammoniumchlorid | 0,5 |
| Panthenol | 0,8 |
| Wasser | @ 100,00 |

Die erfindungsgemäße Entwickler-Kuppler-Kombinationen wurden, unter entsprechender Verringerung des Wassergehalts, in diese Grundlage eingearbeitet.

Die Ausfärbungen erfolgten jeweils an Woll-Läppchen und Strähnen aus gebleichtem Menschenhaar, durch Aufbringen einer 1:1-Mischung aus Farbstoff-Vorprodukt und 6%iger Wasserstoffperoxid-Lösung und zwanzigminütiger Einwirkung bei Zimmertemperatur, folgendem Auswaschen und Trocknen.

Es wurden die folgenden Färbungen erzielt:

| | | |
|---|---|---|
| Beispiel 1: | 0,49 (Gew.-%) | 2-Amino-5-diethylamino toluolhydrochlorid (PC 48) |
| | 0,25 | 3-Aminophenol |
| Färbung: | Graublau. | |

Weitere "PC 48"-Kuppler-Kombinationen und die mit diesen erzielbaren Ausfärbungen werden im folgenden beschrieben:

| | | |
|---|---|---|
| Beispiel 2: | 0,49 (Gew.-%) | PC 48 |
| | 0,25 | Resorcin |
| Färbung: | Helles Purpurgrau. | |

| | | |
|---|---|---|
| Beispiel 3: | 0,49 (Gew.-%) | PC 48 |
| | 0,31 | 2,6-Dihydroxy-3,4-dimethylpyridin |
| Färbung: | Kräftiges Graubeige. | |

| | | |
|---|---|---|
| Beispiel 4: | 0,49 (Gew.-%) | PC 48 |
| | 0,32 | α-Naphthol |
| Färbung: | Kräftiges Mittelblau. | |

| | | |
|---|---|---|
| Beispiel 5: | 0,49 (Gew.-%) | PC 48 |
| | 0,28 | 1-Methyl-2-hydroxy-4-aminobenzol |
| Färbung: | Kräftiges Azurblau. | |

| | | |
|---|---|---|
| Beispiel 6: | 0,49 (Gew.-%) | PC 48 |
| | 0,24 | 1,3-Diaminobenzol |
| Färbung: | Grauviolett. | |

| | | |
|---|---|---|
| Beispiel 7: | 0,49 (Gew.-%) | PC 48 |
| | 0,25 | 2-Amino-3-hydroxypyridin |
| Färbung: | Grauviolett. | |

| | | |
|---|---|---|
| Beispiel 8: | 0,49 (Gew.-%) | PC 48 |
| | 0,25 | 2,6-Diaminopyridin |
| Färbung: | Intensives Hellgrün. | |

| | | |
|---|---|---|
| Beispiel 9: | 0,49 (Gew.-%) | PC 48 |
| | 0,63 | 1-Methoxy-2-amino-4-(β-hydroxyethyl)aminobenzolsulfat |
| Färbung: | Tiefes Graublau. | |

| | | |
|---|---|---|
| Beispiel 10: | 0,49 (Gew.-%) | PC 48 |
| | 0,33 | 4-Chlorresorcin |
| Färbung: | Purpurgrau. | |

| | | |
|---|---|---|
| Beispiel 11: | 0,49 (Gew.-%) | PC 48 |
| | 0,28 | 2-Methylresorcin |
| Färbung: | Glänzendes Braungrau. | |

| | | |
|---|---|---|
| Beispiel 12: | 0,49 (Gew.-%) | PC 48 |
| | 0,25 | 2,-Aminophenol |
| Färbung: | Leuchtendes, intensives Graugrün. | |

| | | |
|---|---|---|
| Beispiel 13: | 0,49 (Gew.-%) | PC 48 |
| | 0,36 | 2,7-Naphthalindiol |
| Färbung: | Helles Braungrau. | |

| | | |
|---|---|---|
| Beispiel 14: | 0,49 (Gew.-%) | PC 48 |
| | 0,31 | 5-Amino-2-methoxyphenol |
| Färbung: | Dunkelblaugrau. | |

| | | |
|---|---|---|
| Beispiel 15: | 0,49 (Gew.-%) | PC 48 |
| | 0,36 | 1,7-Naphthalindiol |
| Färbung: | Mittelblaugrau. | |

| | | |
|---|---|---|
| Beispiel 16: | 0,49 (Gew.-%) | PC 48 |
| | 0,51 | 3-Amino-2-methylamino-6-methoxypyridindihydroclorid |
| Färbung: | Glänzendes, intensives Grautürkis. | |

| | | |
|---|---|---|
| Beispiel 17: | 0,49 (Gew.-%) | PC 48 |
| | 0,41 | 2,6-Diaminopyridindihydrochlorid |
| Färbung: | Glänzendes Milchkaffebraun. | |

| | | |
|---|---|---|
| Beispiel 18: | 0,49 (Gew.-%) | PC 48 |
| | 0,47 | 2-Dimethylamino-5-aminopyridindihydrochlorid |
| Färbung: | Glänzendes, intensives Graumagenta. | |

| | | |
|---|---|---|
| Beispiel 19: | 0,49 (Gew.-%) | PC 48 |
| | 0,36 | 1,5-Diaminonaphthalin |
| Färbung: | Glänzendes Olivbraungrau. | |

| | | |
|---|---|---|
| Beispiel 20: | 0,49 (Gew.-%) | PC 48 |
| | 0,44 | 2,4-Diaminophenolsulfat |
| Färbung: | Glänzendes, intenisves Hellbeigegrau. | |

| | | |
|---|---|---|
| Beispiel 21: | 0,49 (Gew.-%) | PC 48 |
| | 0,28 | 4-Amino-3-methylphenol |
| Färbung: | Spritzige Champagnerfarbe. | |

| | | |
|---|---|---|
| Beispiel 22: | 0,49 (Gew.-%) | PC 48 |
| | 0,39 | 1-Phenyl-3-methylpyrazolon-(5) |
| Färbung: | Rotlila. | |

| | | |
|---|---|---|
| Beispiel 23: | 0,32 (Gew.-%) | PC 48 |
| | 0,18 | 4-Amino-3-methylphenol |
| | 0,18 | 1-Methyl-2-hydroxy-4-aminobenzol |
| Färbung: | Aubergineviolett. | |

| | | |
|---|---|---|
| Beispiel 24: | 0,24 (Gew.-%) | PC 48 |
| | 0,25 | 1-Methyl-2,5-diaminobenzolsulfat |
| | 0,14 | 4-Amino-3-methylphenol |
| | 0,14 | 1-methyl-2-hydroxy-4-aminobenzol |
| Färbung: | Dunkelpurpur. | |

| | | |
|---|---|---|
| Beispiel 25: | 0,39 (Gew.-%) | PC 48 |
| | 0,48 | 3-(Diethylaminomethyl)-4-hydroxyanilindihydrochlorid |
| | 0,11 | 1-Methyl-2-hydroxy-4-aminobenzol |
| Färbung: | Violettgrau. | |

Die folgenden Beispiele 26 bis 32 zeigen, daß auch durch die Kombination von 2-Amino-5-diethylaminotoluol mit Entwicklersubstanzen ohne weiteren Zusatz von Kupplern ausgezeichnete Haarfärbungen erzielt werden.

| | | |
|---|---|---|
| Beispiel 26: | 0,49 (Gew.-%) | PC 48 |
| | 0,50 | 1-Methyl-2,5-diaminobenzolsulfat |
| Färbung: | Glänzender Mittelbraun. | |

| | | |
|---|---|---|
| Beispiel 27: | 0,49 (Gew.-%) | PC 48 |
| | 0,25 | 4-Aminophenol |
| Färbung: | Glänzendes Hellbraun. | |

| | | |
|---|---|---|
| Beispiel 28: | 0,49 (Gew.-%) | PC 48 |
| | 0,54 | 4-Hydroxy-2,5,6-triaminopyrimidinsulfat |
| Färbung: | Glänzendes Beigegelb. | |

| | | |
|---|---|---|
| Beispiel 29: | 0,49 (Gew.-%) | PC 48 |
| | 0,24 | 1,4-Diaminophenol |
| Färbung: | Kräftiges Mittelbraun. | |

| | | |
|---|---|---|
| Beispiel 30: | 0,49 (Gew.-%) | PC 48 |
| | 0,71 | 2-(2'-Hydroxyethylamino)-5-aminotoluolsulfat-3H₂O |
| Färbung: | Glänzendes Hellgraubraun. | |

| | | |
|---|---|---|
| Beispiel 31: | 0,49 (Gew.-%) | PC 48 |
| | 0,56 | 1-β-Hydroxyethyl-2,5- |
| | | diaminobenzolsulfat |
| Färbung: | Glänzendes Hellgraubraun. | |

| | | |
|---|---|---|
| Beispiel 32: | 0,49 (Gew.-%) | PC 48 |
| | 0,66 | (N,N-Bishydroxyethyl)-1,4-diaminobenzolsulfat |
| Färbung: | Glänzendes Graugrün. | |

## Patentansprüche

1. Haarfärbemittel auf Basis eines mit Peroxid reagierenden Oxidations-Farbvorstoffvorprodukt-Systems, dadurch gekennzeichnet, daß es 2-Amino-5-diethylaminotoluol bzw. dessen wasserlösliche Salze enthält.

2. Haarfärbemittel nach Anspruch 1, dadurch gekennzeichnet, daß es 2-Amino-5-diethylaminotoluol bzw. dessen wasserlösliche Salze und mindestens eine Kupplersubstanz enthält.

3. Haarfärbemittel nach Anspruch 2, dadurch gekennzeichnet, daß es mindestens eine Kupplersubstanz, ausgewählt aus der Gruppe Resorcin, 2-Methylresorcin, 4-Chlorresorcin, 2-Amino-4-chlorphenol, 4-(N-Methyl)aminophenol, 2-Aminophenol, 3-Aminophenol, 2,4-Diaminophenol, 5-Amino-2-methoxyphenol, 1-Methyl-2-hydroxy-4-aminobenzol, 3-N,N-Dimethylaminophenol, 4-Amino-3-methylphenol, 5-Amino-2-methylphenol, 6-Amino-3-methylphenol, 5-Amino-2-methoxypyridin, 3-Amino-2-methyl-amino-6-methoxypyridin, 2-Amino-3-hydroxypyridin, 4-Amino-diphenylamin, 4,4'-Diaminodiphenylamin, 2-Dimethylamino-5-aminopyridin, 2,6-Diaminopyridin, 2,5 Diaminopyridin, 2,6-Dihydroxy-3,4-dimethylpyridin, 1,3-Diaminobenzol, 1-Amino-3-(2'-hydroxyethylamino)benzol, 1-Amino-3-[bis(2'-hydroxyethyl)amino]benzol, 1,3-Diaminotoluol, α-Naphthol, 1,4-Diamino-2-chlorbenzol, 4,6-Dichlorresorcin, 4-Hydroxy-1,2-methylendioxybenzol, 1,5-Dihydroxynaphthalin, 1,7-Dihydroxy-naphthalin, 2,7-Dihydroxynaphthalin, 1-Hydroxynaphthalin, 1,5-Diaminonaphthalin, 4-Hydroxy-1,2-methylendioxybenzol, 2,4-Diamino-3-chlorphenol, 1-Phenyl-3-methylpyrazolon-(5), und/oder 1-Methoxy-2-amino-4-(2'-hydroxyethylamino)benzol, enthält.

4. Haarfärbemittel nach einem oder mehreren der Anspüche 1 bis 3, dadurch gekennzeichnet, daß es mindestens eine weitrere Entwicklersubstanz enthält.

5. Haarfärbemittel nach Anspruch 4, dadurch gekennzeichnet, daß es mindestens eine weitre Entwicklersubstanz aus der Gruppe 1,4-Diaminobenzol, 1-Methyl-2,5-diaminobenzol, 2-(2,5-Diaminophenyl)ethanol, 2-(2'-hydroxyethylamino)-5-aminotoluol, N,N-Bis(hydroxyethyl)-1,4-diaminobentol, 3-(Diethylaminomethyl)-4-hydroxyanilin und/oder einen Triaminohydroxypyrimidin bzw. deren wasserlöslichen Salze enthält.

## Claims

1. Composition for the dyeing of human hair on the basis of an oxidation dyestuff precursor system reacting with peroxide, comprising 2-amino-5-diethyl aminotoluene or the water-soluble salts thereof.

2. Composition for the dyeing of human hair according to claim 1, comprising 2-amino-5-diethyl aminotoluene or the water-soluble salts thereof, and at least one coupling agent.

3. Composition for the dyeing of human hair according to claim 2, comprising at least one coupling agent selected from the group resorcinol, 2-methyl resorcinol, 4-chlororesorcinol, 2-amino-4-chlorophenol, 4-(N-methyl)aminophenol, 2-aminophenol, 3-aminophenol, 2,4-diaminophenol, 5-amino-2-methoxy-phenol, 1-methyl-2-hydroxy-4-aminobenzene, 3-N,N-dimethyl aminophenol, 4-amino-3-methyl phenol, 5-amino-2-methyl phenol, 6-amino-3-methyl phenol, 5-amino-2-methoxypyridine, 3-amino-2-methyl amino-6-methoxypyridine, 2-amino-3-hydroxy- pyridine, 4-aminodiphenyl amine, 4,4'-diaminodiphenyl amine, 2-dimethyl amino-5-amino-pyridine, 2,6-diaminopyridine, 2,5-diaminopyridine, 2,6-dihydroxy-3,4-dimethyl pyridine, 1,3-diaminobenzene, 1-amino-3-(2'-hydroxyethylamino)benzene, 1-amino-3-[bis(2'-hydroxyethyl)amino]benzene, 1,3-diaminotoluene, α-naphthol, 1,4-diamino-2-chlorobenzene, 4,6-dichlororesorcinol, 4-hydroxy-1,2-methylenedioxybenzene, 1,5-dihydroxynaphthaline, 1,7-dihydroxynaphthaline, 2,7-dihydroxynaphthaline, 1-hydroxynaphthaline, 1,5-diaminonaphthaline, 4-hydroxy-1,2-methylenedioxybenzene, 2,4-diamino-3-chlorophenol, 1-phenyl-3-methyl pyrazolone-(5), and/or 1-methoxy-2-amino-4-(2'-hydroxyethyl amino)benzene.

4. Composition for the dyeing of human hair according to one or more of claims 1 to 3, comprising at least one additional developing substance.

5. Composition for the dyeing of human hair according to claim 4, comprising at least one additional developing substance selected from the group 1,4-diamino benzene, 1-methyl-2,5-diaminobenzene, 2-(2,5-diaminophenyl)- ethanol, 2-(2'-hydroxyethyl amino)-5-amino toluene, N,N-bis(hydroxyethyl)-1,4-diaminobenzene, 3-diethyl(aminomethyl)-4-hydroxyaniline and/or a triaminohydroxypyrimidine, or the water-soluble salts thereof.

## Revendications

1. Composition de coloration pour cheveux à base d'un système d'un préproduit et d'une présubstance colorante d'oxydation réagissant au peroxyde, caractérisée en ce qu'elle contient du 2-amino-5-diéthylaminotoluène, ou ses sels solubles dans l'eau.

2. Composition de coloration pour cheveux selon la revendication 1, caractérisée en ce qu'elle contient du 2-amino-5-diéthylaminotoluène, ou ses sels solubles dans l'eau, et au moins une substance couplante.

3. Composition de coloration pour cheveux selon la revendication 2, caractérisée en ce qu'elle contient au moins une substance couplante, sélectionnée parmi le groupe composé de résorcine, de 2-méthylrésorcine, de 4-chlororésorcine, de 2-amino-4-chlorophénol,' de 4-(N-méthyl)aminophénol, de 2-amino-phénol, de 3-aminophénol, de 2,4-diaminophénol, de 5-amino-2-méthoxyphénol, de 1-méthyl-2-hydroxy-4-aminobenzène, de 3-N,N,di-méthylaminophénol, de 4-amino-3-méthylphénol, de 5-amino-2-méthylphénol, de 6-amino-3-méthylphénol, de 5-amino-2-méthoxypyridine, de 3-amino-2-méthyl-amino-6-méthoxypyridine, de 2-amino-3-hydroxypyridine, de 4-amino-diphénylamine, de 4,4'-diamino-diphénylamine, de 2-diméthylamino-5-aminopyridine, de 2,6-diaminopyridine, de 2,5-diaminopyridine, de 2,6-dihydroxy-3,4-diméthylpyridine, de 1,3-diaminobenzène, de 1-amino-3-(2'-hydroxy-éthylamino)-benzène, de 1-amino-3-[bis(2'-hydroxyéthyl)amino]-benzène de 1,3-diaminotoluène, d'α-naphtol, de 1,4-diamino-2-chlorobenzène, de 4,6-dichlororésorcine, de 4-hydroxy-1,2-méthylènedioxybenzène, de 1,5-dihydroxynaphtaline, de 1,7-dihydroxy-naphtaline, de 2,7-dihydroxynaphataline, de 1-hydroxy-naphtaline, de 1,5-diaminonaphatline, de 4-hydroxy-1,2-méthylènedioxybenzène, de 2,4-diamino-3-chlorophénol, de 1-phényl-3-méthylpyrazolone-(5), et/ou du 1-méthoxy-2-amino-4-(2'-hydroxy-éthylamino)benzène.

4. Composition de coloration pour cheveux selon une ou plusieurs des revendications 1 à 3, caractérisée en ce qu'elle contient au moins une substance de développement supplémentaire.

5. Composition de coloration pour cheveux selon la revendication 4, caractérisée en ce qu'elle contient au moins une substance de développement supplémentaire issue du groupe composé de 1,4-diaminobenzène, de 1-méthyl-2,5-diaminobenzène, de 2-(2,5-diaminophényl)éthanol, de 2-(2'-hydroxyéthylamino)-5-aminotoluène de N,N-bis(hydroxyéthyl)-1,4-diaminobenzène, de 3-(diéthylaminométhyl)-4-hydroxy-aniline et/ou une triaminohydroxypyrimidine ou ses sels solubles dans l'eau.
